# EUROPÄISCHE PATENTSCHRIFT

(11) **EP 1 153 608 B1**
(45) Veröffentlichungstag und Bekanntmachung des Hinweises auf die Patenterteilung: **17.05.2006**
(21) Anmeldenummer: 01109549.4
(22) Anmeldetag: 18.04.2001
(51) Int. Cl.: A61K 38/36, A61K 38/37, A61K 38/38

(54) **Stabilisiertes Protein-Präparat und Verfahren zu seiner Herstellung**
Stabilised protein preparation and process for the manufacture thereof
Préparation d'une protéine stable et son procédé de fabrication

(30) Priorität: 08.05.2000 DE 10022092
(43) Veröffentlichungstag der Anmeldung: 14.11.2001
(73) Patentinhaber: ZLB Behring GmbH, 35041 Marburg (DE)
(72) Erfinder: Römisch, Jürgen, Dr., 35041 Marburg (DE); Stauss, Harald, 35232 Dautphetal (DE); Stöhr, Hans-Arnold, 35083 Wetter (DE)

(56) Entgegenhaltungen:
- EP-A- 0 230 956
- EP-A- 0 249 167
- EP-A- 0 297 294
- EP-A- 0 383 234
- DE-A- 3 718 889
- US-A- 4 623 717
- US-A- 4 806 524
- US-A- 4 960 757

## Beschreibung

Gegenstand der Erfindung ist ein stabilisiertes Protein-Präparat, das therapeutisch aktive Proteine enthält und durch den Zusatz von Stabilisatoren gegen einen Wirkungsverlust oder die Denaturierung der Proteine bei der Pasteurisierung geschützt ist. Außerdem sind Verfahren zur Virusinaktivierung und Virusabreicherung der erfindungsgemäß stabilisierten Protein-Präparate beschrieben. Hierzu gehören u.a. die Nanofiltration und die Behandlung mit bakteriziden oder viriziden Substanzen oder Detergenzien.

Es ist bekannt, dass bestimmte Proteine in Form von Konzentraten zur Prophylaxe und Therapie unterschiedlicher Krankheiten eingesetzt werden, die durch angeborene oder erworbene Mangelzustände an diesen Proteinen hervorgerufen werden. Als Quelle der therapeutisch angewendeten Proteine dienen dabei besonders das Blutplasma oder Organextrakte. Neuerdings werden auch entsprechende rekombinant oder transgen hergestellte Proteine therapeutisch angewendet.

Jede der genannten Quellen birgt allerdings das potentielle Risiko einer Einschleppung von infektiösen Organismen wie Bakterien, Viren und Prionen. Deshalb sind auch schon zahlreiche Verfahren entwickelt worden, mit denen einer solchen potentiellen Verunreinigung von Protein-Präparaten entgegengewirkt werden kann. Mit der Pasteurisierung, insbesondere der Erhitzung von Proteinlösungen auf 60° C für einen Zeitraum von 10 Stunden, steht bereits ein sehr effektives Verfahren zur Inaktivierung von infektiösen Viren und anderen Krankheitserregern zur Verfügung, das den Sicherheitsstandard hinsichtlich der Übertragung von Infektionen durch Protein-Präparate entscheidend verbessert hat. Zusätzlich wurden weitere Verfahren entwickelt wie die Behandlung der Präparate mit Detergenzien oder bakteriziden oder viriziden Mitteln. Außerdem kann auch eine mechanische Abtrennung von Organismen, bspw. durch eine geeignete Filtration wie die sogenannte "Nanofiltration" erfolgen.

Es reicht jedoch nicht aus, Verfahren zur Verfügung zu stellen, die eine zuverlässige Inaktivierung oder Abreicherung von Viren und anderen pathogenen Keimen in Protein-Präparaten sicherstellen. Gleichzeitig muss auch dafür Sorge getragen werden, dass die therapeutische Wirksamkeit des Proteinpräparats nicht durch Maßnahmen zur Virusinaktivierung oder Virusabreicherung beeinträchtigt wird. Deshalb muss den meist auf konformationelle Veränderungen der Proteine zurückzuführenden Veränderungen durch die Zugabe von Stabilisatoren zu der zu erhitzenden Proteinlösung entgegengewirkt werden. Obwohl Stabilisatoren als Zusatzstoffe zu Protein-Präparaten bereits allgemein Verwendung finden, muss deren qualitative und quantitative Zusammenstellung auf bestimmte Proteine oder Proteingruppen ähnlicher physikochemischer Eigenschaften abgestimmt werden. Dabei finden häufig Kohlenhydrate, nicht selten in Kombination mit bestimmten Aminosäuren, Anwendung.

So ist in der DE-A-29 16 711 ein Verfahren zur Stabilisierung von Blutgerinnungsfaktoren beschrieben, bei dem der Proteinlösung eine Aminosäure und ein Mono- oder Oligosaccharid oder ein Zuckeralkohol zugesetzt werden. Als Aminosäuren werden dabei Glycin, α- oder β-Alanin, Hydroxy-Prolin, Prolin, Glutamin und die α-, β- oder γ-Aminobuttersäure eingesetzt.

Außerdem ist aus den US-Patentschriften 4 440 679 und 4 623 717 bekannt, dass hitzeempfindliche, therapeutisch aktive Proteine wie der Faktor VIII, Fibronektin, Antithrombin III, α-1-Antitrypsin, Plasminogen, Albumin und Prekallikrein ohne Wirkungsverlust pasteurisiert werden können, wenn ihnen entweder eine konzentrierte wässrige Lösung eines Zuckers oder eines reduzierten Zuckers zugesetzt wird, die auch noch 0,1 bis 0,5 Mol/l einer Aminosäure, insbesondere Arginin, Lysin und/oder Glycin enthalten kann.

Schließlich ist auch in der deutschen Patentanmeldung 198 564 43.0 ein stabilisiertes Antithrombin-III-Präparat beschrieben, das gegen einen Wirkungsverlust bei der Pasteurisierung durch einen Zusatz von Stabilisatoren geschützt ist, welche aus einem oder mehreren Sacchariden in Mischung mit mehr als 0,5 mol/l einer oder mehrerer Aminosäuren aus der Gruppe Arginin, Lysin, Histidin, Phenylalanin, Tryptophan, Tyrosin, Asparaginsäure und ihren Salzen oder Glutaminsäure und ihren Salzen bestehen, wobei jeder dieser Aminosäuren auch noch Glycin und/oder Glutamin zugesetzt sein kann.

Es wurde nun gefunden, dass sich nach dem in der deutschen Patentanmeldung 198 564 43.0 beschriebenen Verfahren auch noch andere therapeutisch wertvolle Proteine, die kein Antithrombin III enthalten, so stabilisieren lassen, dass sie eine Pasteurisierung oder ein anderes Verfahren zur Virusinaktivierung oder Virusabreicherung nahezu unbeschadet überstehen. Die nach diesem Verfahren zu stabilisierenden Proteine können sowohl aus Plasma oder Organextrakten hergestellt sein. Insbesondere die Blutgerinnungsfaktoren II, V, VII und Vlla (aktivierte Form von F VII), VIII, IX, X, XII und XIII sowie deren Kombinationspräparate wie das sogenannte "Prothrombinkomplex-Konzentrat", bestehend aus FII, FVII, FIX und FX, und das aktivierte Prothrombinkomplex-Konzentrat sowie Flla (Thrombin) können so mit sehr gutem Erfolg stabilisiert werden. Der gleiche Effekt wurde auch bei dem von Willebrand-Faktor (vWF) oder FVIII/vWF beobachtet.
Dabei lassen sich erfindungsgemäß stabilisierte Protein-Präparate sowohl aus entsprechenden rekombinanten oder transgenen Proteinen herstellen.

Gegenstand der Erfindung ist deshalb ein stabilisiertes Proteinpräparat, das kein Antithrombin III enthält und gegen einen Wirkungsverlust bei der Pasteurisierung durch den Zusatz von Stabilisatoren geschütz ist, welche aus einem oder mehreren Sacchariden in Mischung mit mehr als 0,8 mol/l einer oder mehrerer Aminosäuren aus der Gruppe Arginin, Lysin, Histidin, Phenylalanin, Tryptophan, Tyrosin, Asparaginsäure und ihren Salzen bestehen, wobei eine Aminosäure Glutaminsäure oder eines ihrer Salze sein kann.

Das erfindungsgemäß stabilisierte Protein-Präparat enthält als Saccharid ein Monosaccharid, ein Disaccharid oder ein Oligosaccharid in einer Menge von wenigstens 0,5 g/ml, vorzugsweise von wenigstens 1,5 g/ml. Es weist einen pH-Wert von 3,0 bis 9,5, vorzugsweise von 4,0 bis 8,5 auf. Es enthält außerdem eine oder mehrere der oben genannten Aminosäuren in einer Konzentration von mehr als 0,8 mol/l. Besonders bevorzugt werden Mischungen aus einem Saccharid in einer Konzentration von mehr als 1,5 g/ml mit einer oder mehreren der vorstehend genannten Aminosäuren in Konzentrationen von über 0,8 mol/l. Außerdem ist der Zusatz von löslichen Kalziumsalzen, bspw. in Form von Kalziumchlorid, in Konzentrationen von mehr als 0,5 mmol/l, vorzugsweise mehr als 1 mmol/l, vorteilhaft.

Die so stabilisierte Lösung wird für 5 bis 50 Stunden, vorzugsweise 8 bis 20 Stunden, bei 40 bis 95°C erhitzt, wobei Temperaturen zwischen 50 bis 70°C, insbesondere zwischen 55 bis 65°C, besonders bevorzugt sind. Die erfindungsgemäß stabilisierten Proteinlösungen eignen sich aber auch zur Virusabreicherung mittels Filtration, bevorzugt der Nanofiltration, oder mittels der Zentrifugation oder zur Behandlung mit bakteriziden oder viriziden Mitteln oder mit Detergenzien. Das zuletzt genannte Verfahren ist als "Solvent-Detergent-Behandlung" bekannt.

Die Erfindung wird an folgendem Beispiel erläutert:

### Beispiel

Eine wässrige Proteinlösung, die den Faktor VIII in angereicherter Form enthielt, wurde mit Saccharose bis zu einer Konzentration von 1,75 g/ml versetzt. Diese Lösung wurde in mehrere Ansätze aufgeteilt, denen Glycin oder Glutamat und Arginin beigemischt wurden, um für jede der Aminosäuren Konzentrationen von 0,8 bis 2 mol/l zu erreichen. Die so stabilisierten Lösungen wurden für 10 Stunden bei 60°C im Wasserbad erhitzt. Jedem dieser Ansätze wurde vor und nach dem Erhitzen eine Probe zur Analyse entnommen. Die Faktor VIII-Aktivitäten wurde jeweils nach zwei bekannten Testmethoden untersucht, nämlich dem sogenannten "Clotting Test" und einem chromogenen Test (Coamatic® FVIII). Für jeden Ansatz wurde die Protein-Aktivität berechnet und mit der Aktivität vor der Pasteurisierung verglichen.

Dabei wurde im ersten Ansatz eine Stabilisierung gemäß dem in der DE-A-29 16 711 offenbarten Stand der Technik durchgeführt, während im zweiten Ansatz die erfindungsgemäße Stabilisierung eingesetzt wurde. Die Ergebnisse sind in der nachfolgenden Tabelle dargestellt:

| Ansatz | | Ausbeute (%) |
|---|---|---|
| 1. | Saccharose : 1.75 g/ml | 82 |
| | Glycin : 1.8 mol/l | |
| | CaCl₂ : 0,05 mol/l | |
| | | |
| 2. | Saccharose : 1.75 g/ml | 97 |
| | Na-Glutamat: 1.5 mol/l | |
| | Arginin : 1.5 mol/l | |

Damit wird gezeigt, dass die in der DE-A-29 16 711 beschriebene Stabilisierung mit einem Zucker und einer Aminosäure wie Glycin zwar eine gewisse Stabilisierung des Faktor VIII bewirkt, jedoch zeigt der Ansatz 2, dass durch die erfindungsgemäße Zugabe von Arginin/Glutamat eine erheblich höhere Stabilisierung erreicht wird, die nahezu die gesamte biologische Aktivität des eingesetzten Faktor VIII unbeschadet lässt.

## Patentansprüche

1. Stabilisiertes Protein-Präparat, **dadurch gekennzeichnet,**
**dass** es kein Antithrombin III enthält
und gegen einen Wirkungsverlust bei der Pasteurisierung durch den Zusatz von Stabilisatoren geschützt ist, welche aus einem oder mehreren Sacchariden in Mischung mit mehr als 0,8 mol/l einer oder mehrerer Aminosäuren aus der Gruppe Arginin, Lysin, Histidin, Phenylalanin, Tryptophan, Tyrosin, Asparaginsäure und ihren Salzen oder bestehen, , wobei eine Aminosäure Glutaminsäure oder eines ihrer Salze ist,und dass es als Protein ein oder mehrere Blutgerinnungsfaktoren ausgewählt aus der Gruppe umfassend FII, FV, FVII und FVIIa, FVIII, FIX, FX, FXII sowie deren Kombinationspräparate, den von Willebrand-Faktor (vWF) oder den FVIII/vWF. enthält

2. Stabilisiertes Protein-Präparat nach Anspruch 1, **dadurch gekennzeichnet, dass** es als Saccharid ein Monosaccharid, ein Disaccharid oder ein Oligosaccharid in einer Menge von wenigstens 0,5 g/ml, vorzugsweise von wenigstens 1,0 g/ml, enthält.

3. Stabilisiertes Protein-Präparat nach den Ansprüchen 1 und 2, **dadurch gekennzeichnet, dass** es das Saccharid in einer Menge von mehr als 1,5 g/ml enthält.

4. Stabilisiertes Protein-Präparat nach den Ansprüchen 1 bis 3, **dadurch gekennzeichnet, dass** es zusätzlich auch noch ein lösliches Kalziumsalz in einer Menge von wenigstens 0,5mmol/l, vorzugsweise in einer Menge von wenigstens 1,0 mmol/l enthält.

5. Verfahren zur Virusinaktivierung oder Virusabreicherung eines Protein-Präparates, **dadurch gekennzeichnet, dass** man ein stabilisiertes Protein-Präparat gemäß den Ansprüchen 1 bis 4
- einer Hitzebehandlung bei 40 bis 95°C über einen Zeitraum von 5 bis 50 Stunden oder
- einer Virusabreicherung mittels Filtration oder
- einer Virusabreicherung mittels Zentrifugation oder
- einer Behandlung mit Detergenzien oder bakteriziden oder viriziden Mitteln unterwirft.

## Claims

1. A stabilized protein preparation, which comprises no antithrombin III and is protected against a loss of action during pasteurization by the addition of stabilizers which consist of one or more saccharides as a mixture with more than 0.8 mol/l of one or more amino acids from the group arginine, lysine, histidine, phenylalanine, tryptophan, tyrosine, aspartic acid and its salts, one amino acid being glutamic acid or one of its salts, and where the protein contained is one or more blood clotting factors selected from the group comprising FII, FV, FVII and FVIIa, FVIII, FIX, FX, FXII and their combination preparations, the von Willebrand factor (vWF) or FVIII/vWF.

2. The stabilized protein preparation as claimed in claim 1, **wherein** the saccharide contained is a monosaccharide, a disaccharide or an oligosaccharide in an amount of at least 0.5 g/ml, preferably of at least 1.0 g/ml.

3. The stabilized protein preparation as claimed in claims 1 and 2, **wherein** the saccharide is contained in an amount of more than 1.5 g/ml.

4. The stabilized protein preparation as claimed in claims 1 to 3, **wherein** a soluble calcium salt is additionally also contained in an amount of at least 0.5 mmol/l, preferably in an amount of at least 1.0 mmol/l.

5. A process for the viral inactivation or viral depletion of a protein preparation, **which comprises** subjecting a stabilized protein preparation as claimed in claims 1 to 4
- to a heat treatment at 40 to 95°C for a period of 5 to 50 hours or
- to a viral depletion by means of filtration or
- to a viral depletion by means of centrifugation or
- to a treatment with detergents or bactericidal or virucidal agents.

## Revendications

1. Préparation de protéine stabilisée, **caractérisée en ce qu'**elle ne contient pas d'antithrombine III et est protégée contre une perte d'activité lors de la pasteurisation, par l'addition de stabilisants qui consistent en un ou plusieurs saccharides en mélange avec plus de 0,8 mole/l d'un ou plusieurs aminoacides choisis dans le groupe constitué par l'arginine, la lysine, l'histidine, la phénylalanine, le tryptophane, la tyrosine, l'acide aspartique et leurs sels, un aminoacide étant l'acide glutamique ou l'un de ses sels, et **en ce qu'**elle contient en tant que protéine un ou plusieurs facteurs de coagulation du sang choisis dans le groupe comprenant FII, FV, FVII et FVIIa, FVIII, FIX, FX, FXII ainsi que leurs préparations consistant en associations, le facteur von Willebrand (vWF) ou le FVIII/vWF.

2. Préparation de protéine stabilisée selon la revendication 1, **caractérisée en ce qu'**elle contient en tant que saccharide un monosaccharide, un disaccharide ou un oligosaccharide en une quantité d'au moins 0,5 g/ml, de préférence d'au moins 1,0 g/ml.

3. Préparation de protéine stabilisée selon les revendications 1 et 2, **caractérisée en ce qu'**elle contient le saccharide en une quantité de plus de 1,5 g/ml.

4. Préparation de protéine stabilisée selon les revendications 1 à 3, **caractérisée en ce qu'**en outre elle contient encore un sel de calcium soluble, en une quantité d'au moins 0,5 mmole/l, de préférence en une quantité d'au moins 1,0 mmole/l.

5. Procédé pour l'inactivation de virus ou la diminution de la teneur en virus d'une préparation de protéine, **caractérisé en ce qu'**on soumet une préparation de protéine stabilisée selon les revendications 1 à 4
- à un traitement par la chaleur à une température de 40 à 95°C pendant une durée de 5 à 50 heures ou
- à une diminution de la teneur en virus par filtration ou
- à une diminution de la teneur en virus par centrifugation ou
- à un traitement par des détergents ou des agents bactéricides ou viricides.
